# EUROPEAN PATENT APPLICATION

(11) **EP 4 711 473 A1**
(43) Date of publication of application: **18.03.2026**
(21) Application number: 24199650.3
(22) Date of filing: 11.09.2024
(51) Int. Cl.: C12Q 1/6841

(54) **A METHOD FOR INDEXING A CELLULAR SAMPLE**

(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

A method is provided for indexing a cellular sample (100) comprising the steps: introducing a plurality of nucleic acid barcode elements (108) into at least one cell (102) of the cellular sample (100), and generating a concatenate (104, 202, 206) of at least some of the nucleic acid barcode elements (108) in the at least one cell (102). In a further aspect, a respective kit for indexing the cellular sample (100) is provided.

## Description

### Technical field

The invention relates to a method for indexing a cellular sample with a concatenate of barcode elements. In a further aspect, a respective kit is provided.

### Background

Analysing a cellular sample comprising a large number of individual cells presents several significant challenges, primarily due to the complexity and heterogeneity of the sample. One major issue is the difficulty in distinguishing between different cell types and states within a densely packed tissue section. Traditional bulk analysis techniques often average the signals from all cells, obscuring the unique contributions of rare or distinct cell populations. This loss of resolution can lead to incomplete or misleading interpretations, particularly when trying to understand the underlying mechanisms of diseases like cancer or neurological disorders, where cellular diversity plays a crucial role.

Another problem is the technical and computational challenges associated with handling and processing large datasets generated from single-cell analysis. Techniques such as single-cell RNA sequencing produce large amounts of data, requiring sophisticated algorithms and significant computational resources to manage and analyse effectively. This includes tasks like aligning sequences, quantifying gene expression, and integrating data from multiple samples or experiments. Additionally, the need for high-quality sample preparation and the risk of technical noise or batch effects can complicate the interpretation of results. Ensuring data accuracy and reproducibility in such a high-dimensional context is a non-trivial task, demanding careful experimental design and robust statistical methods to derive meaningful biological insights. Consequently, there is a need to improve existing methods for analysing these cellular samples to enable generating detailed data not only from bulk analyses but from single cell analyses.

### Summary

It is an object to provide a method for securely tracking cells, in particular during analyses of cellular samples.

The aforementioned object is achieved by the subject-matter of the independent claims. Advantageous embodiments are defined in the dependent claims and the following description.

In a first aspect, a method is provided for indexing a cellular sample comprising the steps: introducing a plurality of nucleic acid barcode elements into at least one cell of the cellular sample, and generating a concatenate of at least some of the nucleic acid barcode elements in the at least one cell.

In particular, the cellular sample is a biological sample. The cellular sample may comprise at least two cells. For example, the cellular sample may be a tissue section. Thus, the method may particularly be for indexing individual cells of the cellular sample. In particular, the method may be carried out on a plurality or all of the cells of the cellular sample. This means, a plurality of nucleic acid barcode elements may preferably be introduced at least into a plurality of the cells and a concatenate may be generated in at least the plurality of the cells of the cellular sample. Thus, the elements forming the concatenates may be introduced to the sample and then concatenation may be performed in the sample (in situ). Moreover, several (different) concatenates may be generated in the at least one cell or in any one of the plurality of cells of the cellular sample.

An index may comprise arbitrarily assigned or generated (unique) elements to indicate a particular cell of the cellular sample or to indicate different cells of the cellular sample. By means of the index, the particular cell may be unambiguously identified as that particular cell or a particular one of the different cells may be unambiguously identified as the particular one of the different cells. In particular, this enables tracking the cell throughout an analysis.

The concatenate of the barcode elements may alternatively be referred to as an indexing concatenate and act as an index. By means of the concatenate the cells of the cellular sample may be unambiguously identified. Thus, the method according to the first aspect may be a method for generating indexing elements, such as the concatenate, in the cellular sample.

Each of the nucleic acid barcode elements of the plurality of barcode elements may preferably have a unique nucleotide sequence. In particular, the nucleic acid barcode elements may have a length ranging from 10 to 120 nucleotides. Thus, the nucleic acid barcode elements may differ in their sequence from each other. In particular, there may be multiple copies of each of the unique barcode elements. Each barcode nucleic acid element may be single stranded (a single oligonucleotide) or double stranded (two at least partially complementary oligonucleotides). Further, the sequence of each of the barcode elements may be predetermined. In particular, this means that the sequence of the barcode elements is known when introducing the nucleic acid barcode elements into the cellular sample. For example, a library of barcode elements may comprise 10, 20, 30, 40, 50, 100, 1,000 elements.

The step of introducing the nucleic acid barcode elements may include at least temporarily permeabilising the at least one cell. The concatenate is subsequently generated in the at least one cell in situ. In particular, the step of generating the concatenate may comprise generating at least one or a plurality of concatenates.

Preferably, the method comprises a step of introducing a plurality of anchor elements. Each anchor element is configured to bind to the cellular sample, in particular, each anchor element may be configured to bind specifically to the cellular sample. For example, each anchor element may bind to a particular part or element of the cellular sample or the cell of the cellular sample, such as a genomic DNA locus, or a protein. To that end, the anchor elements may be a nucleic acid partially complementary to the genomic DNA locus or an aptamer, in particular a nucleic acid based aptamer configured to bind to the protein. Alternatively, the anchor elements may be an (amino acid based) antibody (fragment). The anchor elements may additionally be configured to connect to the concatenate of the barcode elements. In particular, the anchor elements may be introduced into the at least one cell prior to generating the concatenate. When generating the concatenate, at least one anchor element may be included in the concatenate. The anchor elements enable fixing the indexing concatenate to the at least one cell. In particular, this enables securely indexing the at least one cell with the concatenate over time. In case the anchor element is configured to bind to a genomic DNA locus, the anchor elements enable fixing the indexing concatenate in a nucleus of the at least one cell of the cellular sample. This may provide a particularly secure way of indexing the at least one cell and reduces the probability of loss of the concatenate from the at least one cell.

It is particularly preferred to generate the anchoring element in a preparatory step by performing an in situ hybridisation of an anchoring probe, which is configured to bind a defined locus or defined number of loci in the genome. To this end ribosomal genes may be particularly useful, but other targets in the genome may be equally suitable. This anchoring probe further comprises an attachment sequence, which serves as an artificially introduced attachment site for concatenate(s). In this way, the number of concatenates forming in the cells may be controlled and there localisation to the nucleus may be achieved, which facilitates segmentation of images (being generated by an optical readout) of the concatenates at a later stage in the workflow, i.e. an isolated bright fluorescent spot in a nucleus, which may be counterstained with DAPI, SYBR Green, Hoechst, or a histone marker like H2B, H2A, or histone H3 antibody-dye conjugates, is easy to detect and segment, which facilitates the readout and decoding of the concatenates that basically represent colour combinations.

Alternatively, or in addition the cellular sample may be infused with precursor molecules configured to form a gel in the sample as it is common practice in the field of expansion microscopy. Such gels may for example be based on naturally occurring or artificial polymers (e.g polyacryl amide) and may be modified to allow cross-linking with the proteins of the sample. This may serve to maintain the relative position of analytes to each other during expansion of the sample by swelling. Such gels may also be used to attach or comprise anchoring elements. The combination of the technology described in this document with the expansion microscopy offers the possibility to image expanded and indexed samples providing super-resolved data acquired on standard widefield microscopes and to combine this data with data derived from downstream single cell multiomics data.

Preferably, the method comprises a step of removing barcode elements that are not part of a concatenate. In particular, un-anchored concatenates may be removed, as well. This step is preferably carried out after generating the concatenate and after introducing the plurality of anchor elements. This enables reducing background noise, for example when reading out the concatenate. For example, the step may comprise washing the cellular sample to remove the barcode element.

Preferably, during the step of generating the concatenate at least some of the barcode elements are ligated together. The ligation may be carried out by a variety of methods, for example by enzymatic ligation, PCR, recombinase-mediated joining, chemical ligation (in particular click chemistry), homologous recombination, rolling circle amplification, or Gibson assembly. In case of a chemical ligation, the nucleic acid barcode elements may comprise respective chemical reaction groups, such as click chemistry groups arranged at respective ends of the nucleic acid barcode elements.

Similarly, the nucleic acid barcode elements may comprise primer sequences or restriction sequences suitable for the particular ligation method.

As an example, the barcode elements may be ligated together with blunt end ligation or with sticky end ligation with all sticky ends having the same complementary sequences. In these examples, the concatenate may be generated randomly from random barcode elements. In an alternative example, each unique barcode element may have sticky ends with a particular sequence. In this alternative example, the concatenate may be generated from predetermined barcode elements. In case of blunt end ligation, the barcode elements may be single stranded. In case of sticky end ligation, the barcode elements may be double stranded. The sticky end ligation may include addition of suitable enzymes, such as restriction enzymes and ligases. In addition, sticky end ligation may include removal of one of the strands of the double stranded barcode elements after ligating the barcode elements.

Preferably, the method comprises a step of introducing a plurality of labels into the at least one cell. Each label comprises a label nucleic acid at least partially complementary to one of the barcode elements, and at least one labelling moiety. The labelling moiety is preferably (covalently) attached to the label nucleic acid. This enables reading out the index, in particular, the indexing concatenate. This step generates a labelled concatenate.

It is preferred to perform the step of concatenate generation using barcode elements comprising sticky ends, which may be generated by digesting the dsDNA barcode elements comprising restriction sites. This also allows to determine the sequence of concatenate formation, i.e. each concatenate may form by concatenating barcode element of set 1, barcode element of set 2,... barcode element of set n. In other words by determining the number of unique elements in barcode element set 1 one can determine, which barcode sequence may appear in a random concatenate at position 1. It is clear that barcode sequence elements may correspond to a colour. For example barcode element set 1 may comprise barcode elements having unique sequences mapped to the following fluorescent dyes ATTO390, ATTO425, ATTO488, ATTO647N. Now the total number of barcode elements forming a concatenate may be undetermined and left to chance or may be predetermined, which can be achieved by selecting appropriate combinations of restriction sites. For example, the concatenates may be predetermined to have only 5 barcode elements corresponding to 5 positions: 1, 2, 3, 4, 5; the positions corresponding to the barcode element sets: 1, 2, 3, 4, 5. Now, in our example the following hypothetical concatenates may form:
Concatenate 1: Position 1 (ATTO488)- ....Position 5()
Concatenate 2: Position 1 (ATTO488)- ....Position 5()
Concatenate 3: Position 1 (ATTO425)- ....Position 5()
Concatenate 4: Position 1 (ATTO647N)- ....Position 5()
Concatenate n: Position 1 (ATTO390)- ....Position 5()

This example shows how each colour represented by a barcode sequence included in barcode element set 1 can randomly be integrated into a concatenate. This essentially generates a random colour code or combination of dyes.

On a high-end spectral confocal imaging system such as STELLARIS 8 (LEICA Microsystems CMS GmbH, Mannheim, Germany) it is possible to separate dyes using spectral information and/or fluorescent lifetime information. In this way panels of dyes comprising 10, 15, 20, 30, 50 or more dyes can be separated by a combination of excitation/emission and lifetime unmixing. The ground plurality of dyes may therefore comprise 10, 15, 20, 30, 50 or more dyes and consequently a large number of possible combinations or permutations of said dyes can be generated.

The labelling moieties may preferably be optically detectable. For example, the labelling moiety may be a fluorophore, such as a fluorescent protein, a fluorescent organic small molecule, or a fluorescent particle such as a quantum dot. Moreover, the labelling moiety may be a molecule with a distinct Raman spectrum, such as a polyyne. The label may comprise a combination of the specific labelling moieties mentioned above. In particular, each label of the plurality of labels comprises at least one labelling moiety that is distinguishable from the remaining labels of the plurality of labels.

The sequence of the label nucleic acid of each label is preferably specific to the labelling moiety of the respective label. For example, the sequence of each label nucleic acid may encode the detectable properties of the respective labelling moiety. Such detectable properties may be optical properties and the labelling moieties may be optically detectable labelling moieties. For example, the detectable properties may include at least one of an excitation or emission wavelength (range), emission lifetime or intensity, or Raman spectrum of the labelling moiety. This enables generating a large number of distinguishable labels.

Since each of the nucleic acid barcode elements of the plurality of barcode elements preferably has a unique nucleotide sequence, each label may be paired with a particular one of the nucleic acid barcode elements. Hence, the sequence of the label nucleic acid of a particular one of the labels may be complementary to a particular one of the nucleic acid barcode elements. Thus, each nucleic acid barcode element may have a particular complementary label, with detectable properties that are distinguishable from other labels. Equally, all labels comprising a particular labelling moiety or a particular combination of labelling moieties with particular detectable properties may comprise a nucleic acid with the identical sequence. In other words, the sequences of the label nucleic acids may uniquely encode the detectable properties of the labelling moieties of the respective label, such that the labelling moieties of a particular label may be identified by the sequence of the nucleic acid of the particular label and in extension by the sequence of the complementary nucleic acid barcode element. In particular, this coding of the detectable properties of the labelling moieties in the label nucleic acid sequence is known when introducing the labels into the cellular sample.

Preferably, an optical readout, in particular an image, is generated of the at least one cell with the labelled concatenate. This enables optically identifying the labelled concatenate of the at least one cell, in particular the detectable properties of the label of the labelled concatenate. The optical readout may be generated by means of a microscope, for example a microscope configured to detect the detectable properties of the labelling moieties. For example, in case of a tissue section, a single image may be generated, or a plurality of images may be stitched together to generate a combined image. Individual cells may be segmented and the respective labelled concatenate may be identified for the at least one cell.

Preferably, based on the optical readout, optical information of the labelled concatenate is determined. The optical information of the labelled concatenate may comprise the detectable properties of the labelling moieties of the labelled concatenate, in particular. For example, individual cells of the cellular sample may be segmented, e.g. by means of image analysis, and the respective labelled concatenate may be identified for the at least one cell. This enables assigning the optical information of the labelled concatenate to the at least one cell, in particular to the optical readout of the at least one cell.

Preferably, the cellular sample is dissociated into individual cells, in particular after generating the optical readout. This enables subsequent analysis of the cells of the cellular sample, in particular of the at least one cell. This step results in a suspension of individual cells. The cellular sample may be dissociated enzymatically or mechanically, for example.

Preferably, the method comprises the step of individualising the at least one cell from the cellular sample, in particular after dissociating the cellular sample. This enables subsequent analysis of the individualised cells. This step may be carried out by means of a microfluidic device with a flow channel, for example.

Preferably, at least some of the individualised cells are encapsulated. This protects the individualised cells and may enable further culturing of the individualised cells, for example. Further, this step may improve handling of the cells. The individualised cells may be encapsulated in a hydrogel, for example.

Preferably, at least a part of the concatenate is amplified. This enables improved detection of the concatenate. The amplification may be achieved by rolling circle amplification or by an isothermal amplification in situ of the at least one cell.

Preferably, the method comprises sequencing of the concatenate generated in the at least one cell and generation of respective sequencing information. This enables detection of the concatenate, in particular determining the specific sequence of the concatenate of the at least one cell. The concatenate, in particular the nucleic acid barcode elements of the concatenate, may comprise primer sequences for sequencing. The step of sequencing the concatenate may include initial addition of primers and enzymes for amplifying the concatenate of barcode elements and subsequently sequencing the amplified concatenates. The step is preferably performed after dissociating and individualising the cells of the cellular sample. Thus, each cell of the cellular sample or a plurality of the cells of the cellular sample may be individualised and their respective concatenates may be individually or separately sequenced. Further, prior to amplifying and/or sequencing of the concatenate, the labels may be removed from the concatenate. The sequencing information may comprise a nucleotide sequence of the concatenate, in particular of the barcode elements of the concatenate. This sequencing information may be assigned to the at least one cell, in particular to the concatenate of the at least one cell.

Preferably, during the step of sequencing of the concatenate a nucleic acid content, such as RNA or genomic DNA, of the at least one cell is sequenced. This enables associating the nucleic acid content of the at least one cell with the indexing concatenate. In particular, the concatenate and the nucleic acid content are sequenced together. This results in a single set of sequence information for the at least one cell, which may comprise sequences of the concatenate and the nucleic acid content of the cell. In particular, the sequencing carried out on the at least one cell is single cell sequencing, preferably, of dissociated and individualised cells. Thus, the sequencing information may be generated for a plurality of the cells of the cellular sample individually for each cell. The sequencing information for each of the cells is therefore separately generated. In particular, the sequencing is carried out after generating the optical readout.

It is particularly preferred, that the optical readout of the at least one cell is assigned to or associated with the sequencing information of the at least one cell based on correlating the sequence of the concatenate, as determined by sequencing the concatenate, to the optical information of the concatenate, as determined from the optical readout. This enables analysing the sequencing information in the particular spatial context of at least one cell within the cellular sample.

Preferably, during the step of sequencing the concatenate a proteomic, epigenomic, or metabolomic analysis of the at least one cell is carried out. The respectively generated analysis information may be associated with or assigned to the sequencing information of the at least one cell; and therefore, with the imaging information. The step is preferably performed after dissociating and individualising the cells of the cellular sample. Thus, each cell of the cellular sample or a plurality of the cells of the cellular sample may be individualised and respective analysis information may be individually or separately generated.

Preferably, each nucleic acid barcode element comprises or consists essentially of a nucleic acid analogue. This enables particular robust nucleic acid barcode elements. For example, this increases the resistance of the nucleic acid barcode elements to degradation agents such as nucleases. Generally, nucleic acid analogues are compounds which are structurally similar to naturally occurring RNA and DNA. Nucleic acids are chains of nucleotides, which are composed of three parts: a phosphate backbone, a pentose sugar, either ribose or deoxyribose, and one of four nucleobases. An analogue may have any of these altered. The nucleic acid analogue may be an artificial nucleic acid or a xeno nucleic acid. Whereas natural nucleic acids or naturally occurring nucleic acids (DNA and RNA) are generally sensitive to degradation agents such as nucleases, nucleic acid analogues are generally resistant to degradation agents such as nucleases. In particular, the nucleic acid barcode elements are resistant to degradation agents such as nucleases. Similarly, the concatenate may comprise or consist essentially of a nucleic acid analogue.

In a further aspect, a kit for indexing a cellular sample is provided. The kit comprises a plurality of nucleic acid barcode elements, and a plurality of labels, which are preferably optically detectable. Each label of the plurality of labels comprises a nucleic acid complementary to one of the barcode elements and at least one labelling moiety, which is preferably optically detectable. The plurality of barcode elements are configured to generate a concatenate when, in particular after, the plurality of barcode elements are introduced into at least one cell of a cellular sample. The plurality of nucleic acid barcode elements and the plurality of labels are configured to carry out the method, in particular as described above.

The kit has the same advantages as the method. Further, the kit may be supplemented with the features of the method described in this document, in particular, the features of the dependent claims of the method.

### Short Description of the Figures

Hereinafter, specific embodiments are described referring to the drawings, wherein:
- Figure 1: is a schematic view of a cellular sample with a concatenate,
- Figure 2: is a schematic view of steps to generate a plurality of concatenates, and
- Figure 3: is a flow chart of a method for indexing a cellular sample.

### Detailed Description

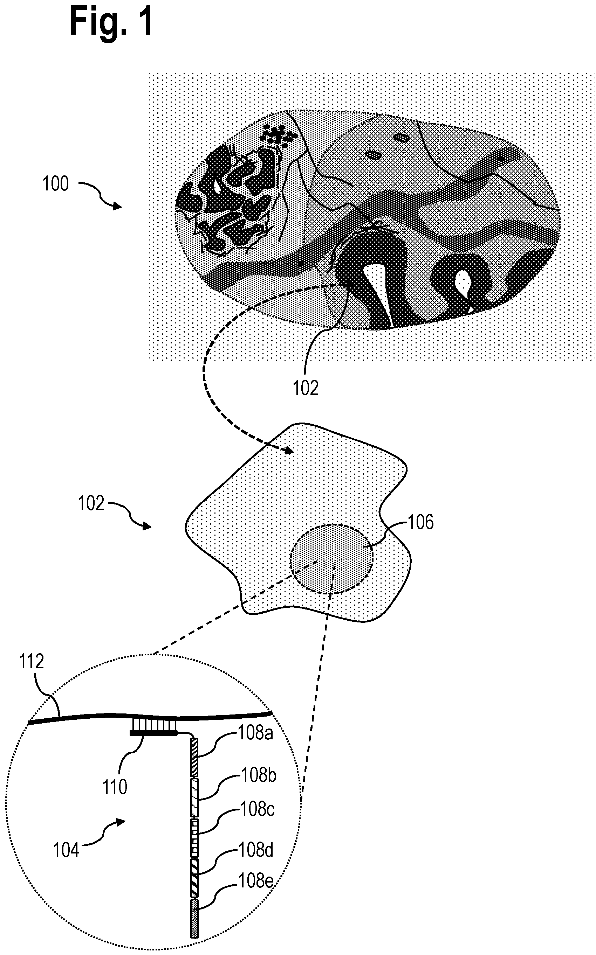

Figure 1 is a schematic view of a cellular sample 100, such as an embedded tissue section, with at least one cell 102 and a concatenate 104 in the at least one cell 102. In particular, the concatenate 104 may be situated in a nucleus 106 of the cell 102. The concatenate 104 comprises a plurality of nucleic acid barcode elements 108a, 108b, 108c, 108d, 108e (collectively referred to with the reference sign 108). The barcode elements 108 each have a unique nucleotide sequence. Thus, each barcode element 108 has a different nucleotide sequence compared to the remaining barcode elements 108. The combined sequence of all the barcode elements 108 of the concatenate 104 acts as a unique identifier for the cell 102.

The barcode elements 108 may be directly connected to each other to generate a chain of the barcode elements 108. For example, this connection may be generated by ligating the individual barcode elements 108 to each other by enzymatic ligation.

Alternatively, the barcode elements 108 may be connected to each other by chemical ligation, for example each barcode element 108 may comprise a first reaction group at a first end and a second reaction group at a second end and the first and second reaction group are configured to react with each other, in particular under specific reaction conditions. For example, the chemical ligation may be based on click chemistry such as a Copper(I)-catalysed azide-alkyne cycloaddition.

In order to securely keep the concatenate 104 within the nucleus 106 of the cell 102, the concatenate 104 comprises an anchor element 110. The anchor element 110 may be a nucleic acid that has a nucleotide sequence complementary to a particular genomic DNA locus of the cell, e.g. the nucleolus 106. Since the genomic DNA 112 is situated in the nucleus 106 of the cell 102, the anchored concatenate 104 is similarly situated in the nucleus 106. The anchor element 110 of the concatenate 104 may be connected to the barcode elements 108 via a linker, such as a linker oligonucleotide.

Alternatively, the anchor element 110 may be an antibody or an amino-acid- or nucleic-acid-based aptamer. Such an anchor element may preferably be configured to bind to a protein of the cell 102, in particular an intracellular protein.

The concatenate 104 may particularly be entirely comprised of nucleotides or be a single nucleic acid molecule. In a particular embodiment, the barcode elements 108, in particular the concatenate 104, may comprise a nucleic acid analogue.

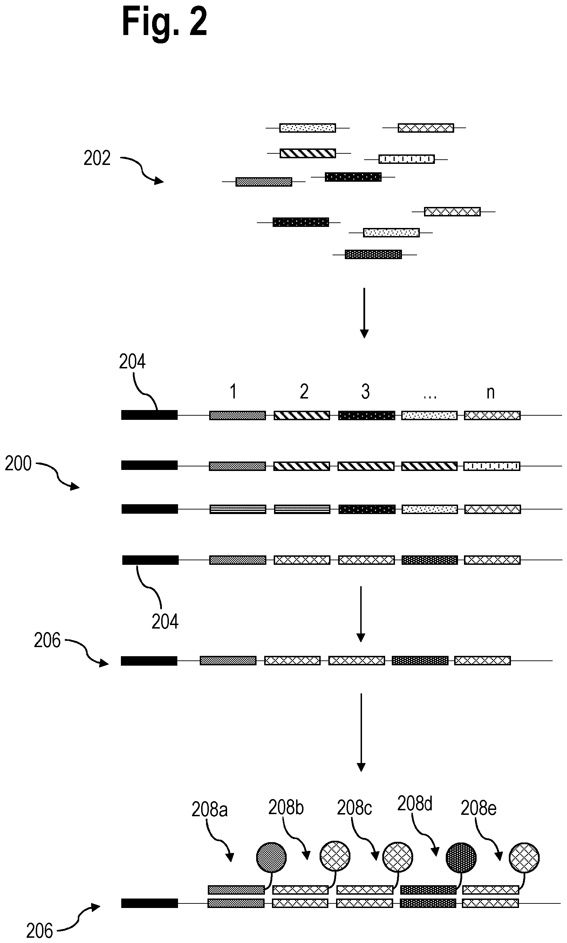

Figure 2 is a schematic view of steps to generate a plurality of concatenates 200 from a plurality of individual nucleic acid barcode elements 202. The barcode elements 202 may initially be introduced into a cell of a cellular sample, for example after permeabilising cell membranes of the cells of the cellular sample. The barcode elements 202 may be randomly ligated to each other within the cell, for example by enzymatic ligation.

The enzymatic ligation may be a blunt end ligation, for example. Alternatively, sticky end ligation may be used, in which case the barcode elements 202 are provided as double stranded nucleic acid molecules. The sticky ends are preferably generated by addition of restriction enzymes after the barcode elements 202 are introduced into the cell of the cellular sample. The sticky ends of each barcode element 202 may all be identical. Thus, ligating the barcode elements 202 together may result in a plurality of concatenates 200 with a random order or barcode elements 202 and/or a random number of n barcode elements 202.

Optionally, the concatenates 200 may comprise anchor elements 204, which may have a nucleotide sequence complementary to a particular genomic nucleic acid of the cell 102, for example. The anchor elements 204 may be ligated to the barcode elements 202 similarly as described for the barcode elements 202 above.

Depending on the number of the barcode elements 202 and the number of nucleotides of each barcode element 202, a large number of concatenates 200 with unique and distinguishable nucleotide sequences may be generated. This unique sequence property of the concatenates 200 may be used to index the cell that contains the concatenates 200. In particular, concatenates 200 may be generated in each cell of a cellular sample in order to index each of the cells. By randomly generating the concatenates 200 in each cell from the plurality of barcode elements 202, each cell has a particular combination of concatenates 200 with a particular permutation of the individual barcode elements 202. This unique combination and/or permutation enables indexing a large number of cells. The index of the cells may be used to identify each cell by the nucleotide sequence of the concatenates 200 contained in the particular cell and therefore by the order of the barcode elements 202 of the concatenates 200.

In order to identify each cell, single-cell sequencing may be performed during which the sequence of the concatenates 200 may be determined.

The concatenates 200 and therefore the index of the cells may further be identified optically. In particular, by labelling each concatenate 200 with labels. This is exemplified for a concatenate 206 of the concatenates 200.

A plurality of labels 208a, 208b, 208c, 208d, 208e (collectively referred to by reference sign 208) may be introduced into the cell that contains the concatenates 200 in order to be able to optically read out the concatenates 200. This may include generating an optical readout, in particular an image of the cell containing the concatenates 200, for example by means of a microscope. Each of the labels 208 may comprise at least one labelling moiety and a label nucleic acid with a nucleotide sequence complementary to one of the barcode elements 202. Each label with a label nucleic acid that is complementary to a particular one of the barcode elements 202 has a labelling moiety with identical detectable properties. For example, the labelling moieties may be fluorophores, and the detectable properties may include excitation or emission wavelength, emission lifetime, or emission intensity. Thus, the nucleotide sequence of the concatenates 200 may be determined optically from the detectable properties of the labels 208 attached to the concatenates 200.

For unambiguous identification of the concatenates 200 it may be enough to determine the combination of labels 208 attached to the concatenates 200. It may not be necessary to determine the particular order or permutations of the labels 208 attached to the concatenates 200.

In case the concatenates 200 contained in a cell are identified based on the detectable properties of the labels 208 a subsequent (single cell) sequencing of the cell including the concatenates 200 enables linking the generated sequencing information e.g. of a nucleic acid content of the cell, to a particular location or context of the cell within the original cellular sample.

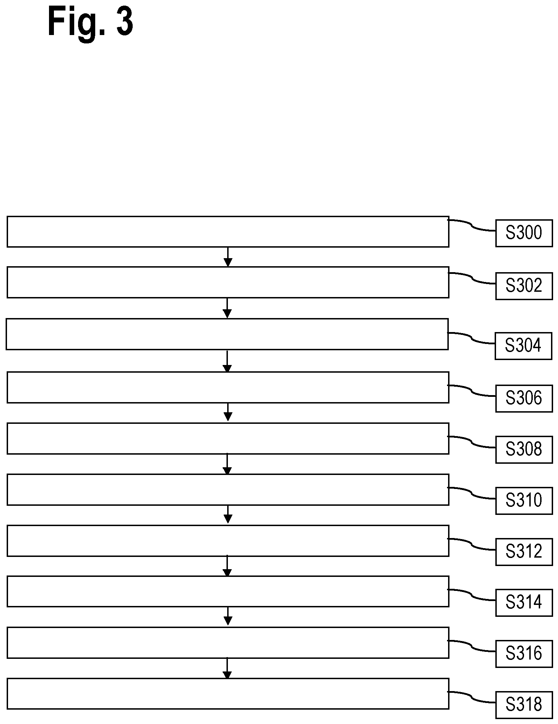

Figure 3 is a flow chart of a method for indexing a cellular sample. The method starts with step S300. In step S302, a plurality of nucleic acid barcode elements are introduced into at least one cell of the cellular sample. This may include permeabilising the at least one cell. Preferably, the plurality of nucleic acid barcode elements are introduced in a plurality of the cells of the cellular sample. The plurality of nucleic acid barcode elements comprises multiple copies of barcode elements with unique nucleotide sequences. In particular, step S302 may partially follow a FISH protocol for introducing nucleic acid based FISH probes into cells. Optionally, an anchor element may be introduced into in step S302.

In step S304, the concatenates of the nucleic acid barcode elements are generated in the cells. Optionally the concatenates may be generated to include the anchor elements. For example, the nucleic acid barcode elements may be ligated together such that a chain of nucleic acid barcode elements is generated. This may be carried out in a random fashion, for example based on blunt end ligation, in order to generate random combinations, in particular permutations, of the barcode elements. In an alternative, the nucleic acid barcode elements may be ligated by sticky end ligation.

In an optional step S306, any barcode elements not concatenated in a concatenate may be removed, for example by washing the cellular sample. Further, this step may include removing concatenates that have not anchored to the cell, despite anchor elements previously introduced into the cell.

The method continues with step S308, in which labels may be introduced into the cell. The labels comprise label nucleic acids, in particular a barcode sequence, complementary to a respective one of the nucleic acid barcode elements previously introduced into the cell. Each label further comprises a labelling moiety that has detectable properties. In particular, the assignment of the particular labelling moiety to the particular (unique) label nucleic acid is known or pre-determined, e.g. when the labels are generated. Each label with a particular nucleotide sequence has a labelling moiety with detectable properties that are distinguishable from the labelling moiety of a label with a different nucleotide sequence. Due to the complementarity between the label nucleic acids and barcode elements, the labels bind to their respective barcode elements of the concatenates after being introduced to the cell. This enables detecting and identifying the concatenates via the labels.

In an optional step S310, unbound or excess labels may be removed from the cellular sample, for example by washing the sample. In particular, this reduces background noise due to unbound labels.

In a step S312, an optical readout, such as an image, of the cellular sample, in particular of the cell, may be generated. For example, the optical readout may be generated by means of a microscope. In particular, the microscope may be configured to detect the detectable properties of the labels. The concatenates, in particular the respective labels attached to the concatenates, may be identified in the optical readout. The detectable properties of each of the identified concatenates may be determined in the optical readout and assigned to the respective cell they are in as optical information. To that end, the optical readout may be segmented and individual cells with their respective concatenates may be identified in the segmented cells.

In a step S314, the concatenates of the cell is sequenced. In particular, the nucleotide sequence of the barcode elements of each concatenate of the cell is determined. Based on this respective sequencing information may be generated. This step may optionally include sequencing of a nucleic acid content of the cell, for example, the genomic DNA and/or the RNA of the cell. The nucleotide sequences of the nucleic acid content of the cell may similarly be included in the sequencing information.

Preferably, the sequencing in step S314 is preceded by dissociating the cellular sample into individual cells and individualising the cell from the dissociated cellular sample. Thus, the sequencing of step S314 may be single cell sequencing of the cell of the cellular sample. Thus, the sequencing information may only comprise nucleotide sequences determined from the cell, rather than the entire cellular sample. This enables securely assigning the sequencing information only to the cell.

Alternatively or in addition to sequencing of the genetic content of the cell, further analyses such as proteomic, epigenomic and/or metabolomic analyses may be carried out in step S314. Respective analysis information may be included in the sequencing information.

In a step S316, the optical readout of the (segmented) cell of step S312 is assigned to the sequencing information of the step S314 based on correlating the nucleotide sequence of the concatenates determined in step S314 to the optical information of the concatenate determined in step S312. In particular, this is based on each label with a particular labelling moiety being complementary to a particular one of the barcode elements. This enables determining which combination or permutation of detectable properties in the optical readout correlates to which combination or permutation of barcode element sequences in the sequencing information.

The method ends in step S318.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100: Cellular sample
- 102: Cell
- 104, 202, 206: Indexing concatenate
- 106: Nucleus of cell
- 108, 108a, 108b, 108c, 108d, 108e, 202: Nucleic acid barcode elements
- 110, 204: Anchor element
- 112: Genomic DNA
- 208, 208a, 208b, 208c, 208d, 208e: Label

## Claims

1. A method for indexing a cellular sample (100) comprising the steps:
introducing a plurality of nucleic acid barcode elements (108) into at least one cell (102) of the cellular sample (100), and
generating a concatenate (104, 202, 206) of at least some of the barcode elements (108) in the at least one cell (102).

2. The method according to claim 1, comprising the step of introducing a plurality of anchor elements (110, 204), wherein each anchor element (110, 204) is configured to bind to the cellular sample (100).

3. The method according to one of the preceding claims, comprising the step of removing barcode elements (108) that are not part of the concatenate (104, 202, 206).

4. The method according to one of the preceding claims, wherein during the step of generating the concatenate (104, 202, 206) at least some of the barcode elements (108) are ligated together.

5. The method according to one of the preceding claims, comprising the step of introducing a plurality of labels (208) into the at least one cell (102), wherein each label (208) comprises a nucleic acid complementary to one of the barcode elements (108), and at least one labelling moiety.

6. The method according to claim 5, wherein an optical readout is generated of the at least one cell (102) with the labelled concatenate (104, 202, 206).

7. The method according to claim 6, wherein based on the optical readout, optical information of the labelled concatenate (104, 202, 206) is determined.

8. The method according to one of the preceding claims, wherein the cellular sample (100) is dissociated into individual cells (102).

9. The method according to one of the preceding claims, wherein the method comprises the step of individualising the at least one cell (102) from the cellular sample (100).

10. The method according to claim 9, wherein at least some of the individualised cells (102) are encapsulated.

11. The method according to one of the preceding claims, wherein at least a part of the concatenate (104, 202, 206) is amplified.

12. The method according to one of the preceding claims, comprises sequencing of the concatenate (104, 202, 206) generated in the at least one cell (102) and generation of respective sequencing information.

13. The method according to claim 12, wherein during the step of sequencing of the concatenate (104, 202, 206) a nucleic acid content of the at least one cell (102) is sequenced.

14. The method according to one of the preceding claims 12 and 13, wherein the optical readout of the at least one cell (102) is assigned to the sequencing information of the at least one cell (102) based on correlating the sequence of the concatenate (104, 202, 206) to the optical information of the concatenate (104, 202, 206).

15. The method according to one of the preceding claims 12 to 14, wherein during the step of sequencing the concatenate (104, 202, 206) a proteomic, epigenomic, or metabolomic analysis of the at least one cell (102) is carried out.

16. A kit for indexing a cellular sample (100) comprising
a plurality of nucleic acid barcode elements (108), and
a plurality of labels (208), and
wherein each label (208) of the plurality of labels (208) comprises a nucleic acid complementary to one of the barcode elements (108) and at least one labelling moiety,
wherein the plurality of barcode elements (108) is configured to generate a concatenate (104, 202, 206) when the plurality of barcode elements (108) is introduced into at least one cell (102) of a cellular sample (100), and
wherein the plurality of nucleic acid barcode elements (108) and the plurality of labels (208) are configured to carry out the method according to one of the previous claims.
